# EUROPÄISCHE PATENTSCHRIFT

(11) **EP 1 550 716 B1**
(45) Veröffentlichungstag und Bekanntmachung des Hinweises auf die Patenterteilung: **04.03.2009**
(21) Anmeldenummer: 04030534.4
(22) Anmeldetag: 23.12.2004
(51) Int. Cl.: C12N 5/00

(54) **Verfahren zur Herstellung dreidimensionaler trägerfreier Gewebestrukturen und nach diesem Verfahren hergestellte Gewebestrukturen**
Manufacturing process for three-dimensional tissue structures und structures obtainable thereby
Procédé pour la fabrication de structures de tissu tridimensionnelles, et structures de tissu fabriqués avec ce procédé

(30) Priorität: 05.01.2004 DE 102004001225
(43) Veröffentlichungstag der Anmeldung: 06.07.2005
(73) Patentinhaber: fzmb Forschungszentrum für Medizintechnik und Biotechnologie e.V., 99947 Bad Langensalza (DE)
(72) Erfinder: Wilke, Ingo, 37133 Friedland (DE); Ponomarev, Igor, 99876 Gotha (DE)
(74) Vertreter: Bock, Gerhard

(56) Entgegenhaltungen:
- WO-A1-97/49799
- WO-A2-02/33052
- US-A- 5 153 136
- NIKLASON L E ET AL: "Functional arteries grown in vitro" SCIENCE, AMERICAN ASSOCIATION FOR THE ADVANCEMENT OF SCIENCE,, US, Bd. 284, Nr. 5413, 16. April 1999 (1999-04-16), Seiten 489-493, XP002198665 ISSN: 0036-8075
- SODIAN R ET AL: "NEW PULSATILE BIORECTOR FOR FABRICATION OF TISSUE-ENGINEERED PATCHES" JOURNAL OF BIOMEDICAL MATERIALS RESEARCH, WILEY, NEW YORK, NY, US, Bd. 58, Nr. 4, 2001, Seiten 401-405, XP001037742 ISSN: 0021-9304

## Beschreibung

Die vorliegende Erfindung betrifft ein Verfahren zur Herstellung dreidimensionaler trägerfreier Gewebestrukturen, insbesondere von Knochen- oder Knorbelzellen, sowie die nach diesem Verfahren hergestellten Gewebestrukturen.

Seit einiger Zeit ist bekannt, dass Zellkulturen von tierischen und menschlichen Zellen kultivierbar sind, wobei die Zellen als Monolayer auf Trägersubstrate aufwachsen bzw. dreidimensionale Gebilde in dem sie umgebenden Nährmedium oder auf Trägern ausbilden.
Die Vereinzelung bzw. Suspension der Zellen dieser Kulturen erfolgt durch mechanische und/oder enzymatische Behandlungen (Toni Lindl, "Zell- und Gewebekultur", Spektrum Verlag, 4. Auflage, Seite 97-101).
Die Schrift DE 199 62 456 A1 offenbart ein Verfahren und eine Apparatur zur Herstellung, Untersuchung und Simulation von dreidimensionalen Zellkulturen sowie einen in-vitro Testsystem von medizinischen Wirkstoffen. Die Zellen werden dabei in eine schützende Flüssigkeit, bspw. Hyaloronsäure, gebracht und bspw. durch ein poröses Material abgepresst, wobei eine zyklische oder permanente mechanische Belastung erfolgt. Die Versorgung der Zellen wird dabei durch eine Membran, einen Filter oder ein poröses Sintermaterial durch Diffusion gewährleistet, so dass ein Aufwachsen der Zellen auf den Träger erfolgt.

Das Problem dieses Verfahrens ist, dass das verwendete Trägermaterial nicht vollständig biologisch resorbierbar ist und die Zellen für ihre weitere Verwendung, bspw. Transplantation, von dem Trägermaterial mechanisch oder enzymatisch abgelöst werden müssen, um den Träger nicht mit zu implantieren.

Der Erfindung liegt daher die Aufgabe zu Grunde, ein Verfahren zur Herstellung dreidimensionaler trägerfreier Gewebestrukturen anzugeben, das ohne mechanische oder enzymatische Ablösung der Zellstrukturen von Trägermaterialen und ohne Verwendung von Cytokinen oder anderen bioaktiven Zusatzstoffen auskommt.

Diese Aufgabe wird durch ein Verfahren gemäß Anspruch 1 gelöst. Vorteilhafte Ausgestaltungen des erfindungsgemäßen Verfahrens werden in den nachgeordneten Ansprüchen angegeben.

Das Wesen der Erfindung besteht darin, dass durch die einzelnen Schritte des Verfahrens aus eukaryotischen Zellen mechanisch konditionierte Flächen oder dreidimensionale Gebilde von Knochenoder Knorbelzellen entstehen, die direkt zur Implantation eingesetzt werden können.

Die Erfindung soll nachfolgend des Ausführungsbeispiels näher erläutert werden.

### Vorbereitung, Anzucht und Kultivierung

Isolierte Zellen aus Knorpel, Knochen, Knochenmarksaspiraten, Nucleus pulposus oder des Anulus fibrosus werden in Adhäsionsmedium aufgenommen. Das Adhäsionsmedium dient zur ersten Adhäsion von Zellen an den Boden der Kulturgefäße. 5-10x10³ Zellen/cm² Knorpel-, Knochen-, Bandscheiben-Zellen oder aus Knochenmark (2x10⁵ Zellen/cm²⁾ isolierten Zellen werden in 75-cm² Zellkulturflaschen überführt. Zur Adhäsion oben genannter Zellen wird handelsübliches DMEM "low glucose" Medium (10% FCS, 100 U Penicillin/ml, 100 µg Streptomycin/ml) verwendet.

Nach der Adhäsion der Zellen an den Boden der Kulturflasche wird ein anderes Zellkulturmedium verwendet.
Als Kulturmedium wird eine 1/1 Mischung aus DMEM "high glucose" und Ham's F12 verwendet, welches mit folgenden weiteren Zusätzen supplementiert wird: 10% FCS, 50 µg Ascorbinsäure/ml, 30 µg α-Ketoglutarsäure/ml, 100 U Penicillin/ml und 100 µg Streptomycin/ml. Die Kultivierung erfolgt im Brutschrank mit 90% Feuchtigkeit, 5% CO₂ bei 37°C. Der Mediumwechsel erfolgt alle drei bis vier Tage.

Nach Erreichung der 100%igen Konfluenz müssen die Monolayerkulturen noch zwei bis fünf Tage "nachreifen". Am sechsten Tag wird das Medium entfernt und der Monolayer mit 2x10 ml steriler PBS Lösung (pH 7.4) ohne Ca und Mg Supplementierung gespült. Zu jeder Flasche werden 15 ml der gleichen Lösung zugegeben und für 30-90 Minuten in einem Brutschrank (90% Feuchtigkeit, 5% CO₂, 37°C) inkubiert. Während dieser Zeit (visuelle Kontrolle) löst sich der Monolayer vom Kulturgefäß-Boden ab. Nach vollständiger Ablösung wird das sich in der Suspension befindliche Monolayer-Zell-Vlies mittels einer 10 ml Pipette abgesaugt (vorsichtig) und in sterile 50 ml Röhrchen übertragen. Anschließend werden 10 ml Kultivierungs-Medium zugegeben (vorsichtig, dass das Vlies nicht zerstört wird) und bei 100 bis 1000g 10 Minuten zentrifugiert.
Danach wird der Überstand entfernt und vorsichtig 5 ml frisches Kulturmedium auf das Pellet gegeben. Das Röhrchen wird nicht vollständig geschlossen und senkrecht in den Brutschrank gestellt. Nach ein bis zwei Tagen bildet sich aus dem Pellet ein deutlich sichtbarer kugelförmiger Cluster. In diesem Zustand wird der Cluster bis zu sieben Tagen gereift, mit täglichem Mediumwechsel (Kultivierungsmedium, 5ml). Danach wird der Cluster mittels sterilen Löffels in eine Vertiefung einer 6er Multiwell-Platte übertragen.

### Erste Druck-Belastung

Die erste Druckbelastung wird nach der Übertragung des Clusters durchgeführt. Diese erste Druckbelastung erfolgt mit einem Glaspistill mit glatter, scheibenförmiger Oberfläche (Durchmesser von 1 bis 3cm²). Unter erster Druckbelastung verstehen wir die Komprimierung des Clusters bis er etwa 50% seiner Anfangsgröße einnimmt. Die Druckfrequenz liegt bei 0.5 Hz, die Belastungszeit zwischen 15 und 25 Sekunden. Nach dieser Belastung sieht dieser Cluster wie ein "Pfannkuchen" aus. Hierauf wird sofort das Medium (vorsichtig!) entfernt. Danach wird der Cluster wie eine Apfeltasche gefaltet und umgedreht. Anschließend werden tropfenweise 2 ml Kulturmedium pro Cluster zugegeben. Die Weiterkultivierung erfolgt im Brutschrank.

### Weitere Druck-Belastungen

Am zweiten Tag wird die Druckbelastung 2 x durchgeführt (0.5 Hz, 15-25 sec). Anschließend wird das "Apfeltaschenverfahren" wiederholt.
Am dritten Tag erfolgt eine dreimalige Belastung (0.5 Hz, 15-25 sec), mit Wiederholung der "Apfeltaschenherstellung". Dieses Verfahren wird bis zum 5ten Kultivierungstag wiederholt (die "Apfeltaschenherstellung" kann schon vor dem 5. Kultivierungstag gestoppt werden, wenn der Cluster in einer stabilen gelartigen Form bleibt).
Ist diese dreidimensionale Zellstruktur entstanden bzw. stabil geworden, kann die Weiterkultivierung (Druckbelastung) manuell oder mit einem dafür vorgesehenen Druckreaktor fortgesetzt werden.
Nach Erreichung der stabilen gelartigen Cluster-Form kann der Druck stufenförmig erhöht werden bis 10x pro Tag (0.5Hz, 15-25 sec, 10-500 g/cm²).
Nach 12 bis 20 Belastungs-Tagen erreicht der Cluster (im Versuch) eine genügende Festigkeit, um dieses so "in vitro" gewonnene Material für Transplantationsversuche oder autologe Transplantationen zu verwenden.

### Verwendung der dreidimensionalen Zellstruktur:

Das mit dem erfindungsgemäßen Verfahren regenerierte dreidimensionale Zellsystem kann als Modell- und Untersuchungssystem sowie für Transplantationen verwendet werden.
Weiterhin dient die Struktur als Modell zur Überprüfung der Wirksamkeit von Pharmazeutika. Zusätzlich eignet sich dieses dreidimensionale Regenerat als Untersuchungsmodell und Release-System für gen- und zelltherapeutische Einsätze von spezifischen Transportvehikeln wie "VLP" basierte Systeme aber auch für adenovirale und retrovirale Transportsysteme.

## Patentansprüche

1. Verfahren zur Herstellung dreidimensionaler trägerfreier Gewebestrukturen bei dem
- aus Knorpel oder Knochen oder Knochenmarksaspiraten oder Nucleus pulposus oder des Anulus fibrosus isolierte Zellen in einem Adhäsionsmedium auf einen festen Träger aufwachsen,
- diese aufgewachsenen Zellen zwei bis fünf Tage unter periodischem Medienwechsel zu einer Zellschicht reifen,
- im Anschluss daran diese aufgewachsenen Zellschicht mit Ca²⁺-und Mg²⁺-ionenfreiem Zellkulturpuffer mindestens einmal gespült werden,
- um dann 30 bis 90 Minuten in einem Brutschrank in diesem Puffer inkubiert zu werden, wobei sich die Zellschicht von dem Träger ablöst,
- die abgelöste Zellschicht in Zellkulurmedium überführt und mechanisch abgeschieden wird, so dass ein Zellhaufen entsteht,
- dieser Zellhaufen bis zu sieben Tage mit periodischem Mediumwechsel inkubiert wird, so dass einen Zellcluster heranreift,
- und dieser Zellcluster im Anschluss an diese Inkubation jeweils mindestens einer zyklischen Druckbelastung mit anschließender mechanischer Formgebung unterworfen wird.

2. Verfahren zur Herstellung dreidimensionaler trägerfreier Gewebestrukturen nach Anspruch 1, **dadurch gekennzeichnet, dass** der erste Schritt der zyklischen Druckbelastungen zu einem Zellcluster führt, der auf bis zu 50% seiner Größe vor der Druckbehandlung reduziert ist, und die mechanische Formgebung durch einmaliges Falten des Zellclusters erzeugt wird.

3. Verfahren zur Herstellung dreidimensionaler trägerfreier Gewebestrukturen nach Anspruch 2, **dadurch gekennzeichnet, dass** die Druckbelastung mit einer Druckfrequenz von 0.5 Hz und einer Belastungszeit von 15 bis 25 Sekunden durchgeführt wird.

4. Verfahren zur Herstellung dreidimensionaler trägerfreier Gewebestrukturen nach Anspruch 1, **dadurch gekennzeichnet, dass** die zweiten und weiteren Schritte der zyklischen Druckbelastungen durch jeweils zeitlich zwischengelagerte mechanische Formgebung durch einmaliges Falten des Zellclusters unterbrochen werden.

5. Verfahren zur Herstellung trägerfreier dreidimensionaler Gewebestrukturen nach Anspruch 5, **dadurch gekennzeichnet, dass** die Druckbelastung mit einer Druckfrequenz von 0.5 Hz und einer Belastungszeit von 15 bis 25 Sekunden durchgeführt wird.

6. Verfahren nach einem oder mehreren der voranstehenden Ansprüche, **dadurch gekennzeichnet, dass** die zyklische Druckbelastung mit anschließender mechanischer Formgebung nach dem 5. Kultivierungstag abgebrochen wird und der Zellkluster bis 10 mal pro Tag mit einer Druckfrequenz von 0.5Hz, einer Belastungszeit von 15-25 sec mit 10 bis 500 g/cm² über einen Zeitraum von bis zu 20 Tage belastet wird.

7. Dreidimensionale trägerfreier Gewebestrukturen hergestellt nach dem Verfahren gemäß Anspruch 1.

## Claims

1. Process for manufacturing three-dimensional carrier-free tissue structures, and in said process
- cells isolated from cartilage or bones or bone marrow aspirates or nucleus pulposus or anulus fibrosus grow on a fixed carrier in adhesion medium,
- said cultivated cells mature to one cell layer with a periodic medium change for two to five days,
- afterwards said cultivated cell layer is purged with Ca²⁺- and Mg²⁺-ion-free cell culture buffer at least once,
- to be incubated in this buffer in an incubator for 30 to 90 minutes then, and during this process the cell layer detaches from the carrier,
- the detached cell layer is added to a cell culture medium and mechanically separated so that a cell crowd develops,
- said cell crowd is incubated with a periodic medium change for up to seven days so that a cell cluster matures,
- and following this incubation said cell cluster is subject to at least one cyclie compression load with subsequent mechanical shape forming.

2. Process for manufacturing three-dimensional carrier-free tissue structures as set forth in claim 1, wherein the first step of the cyclic compression load leads to a cell cluster that is reduced to 50% of the size it had before the compression load and the mechanical shape forming is carried out by folding the cell cluster once.

3. Process for manufacturing three-dimensional carrier-free tissue structures as set forth in claim 2, wherein the compression load is performed at a pressure frequency of 0.5 Hz and during a load time of between 15 and 25 seconds.

4. Process for manufacturing three-dimensional carrier-free tissue structures as set forth in claim 1, wherein the second and further steps of the cyclic compression loads are always interrupted by mechanical shape forming processes that are fitted in between said loads and are performed by folding the cell cluster once.

5. Process for manufacturing three-dimensional carrier-free tissue structures as set forth in claim 5, wherein the compression load is performed at a pressure frequency of 0.5 Hz during a load time of between 15 and 25 seconds.

6. Process as set forth in one or several of the previous claims, wherein the cyclic compression loads with subsequent mechanical shape forming is terminated after the fifth day of cultivation and the cell cluster is loaded with 10 bis 500 g/cm² at a pressure frequency of 0.5 Hz during a loading time of between 15 and 25 sec up to ten times a day over a period of up to 20 days.

7. Three-dimensional carrier-free tissue structures manufactured according the process described in one or several of the previous claims.

## Revendications

1. Procédé pour la fabrication de structures de tissu tridimensionnelles pour lesquelles
- des cellules isolées consistant en cartilage ou os ou moelle osseuse gagnée par ponction-aspiration ou Nucleus pulposus ou Anulus fibrosus croissent dans un milieu d'adhésion sur un support solide,
- ces cellules crues mûrissent avec un changement de milieu périodique pendant deux à cinq jours pour former une couche des cellules,
- à la suite de cette maturité, cette couche des cellules crue est rincée une fois au minimum avec un tampon de culture cellulaire exempt d'ions Ca²⁺ et Mg²⁺,
- pour être incubé ensuite avec ce tampon dans un incubateur pendant 30 à 90 minutes, ce qui mène à la séparation de la couche des cellules du support,
- la couche des cellules détachée est transférée dans le milieu de culture cellulaire et séparée mécaniquement de sorte qu'un amas cellulaire se forme,
- cet amas cellulaire est incubé pendant une durée de jusqu'à 7 jours en changeant périodiquement le milieu de sorte qu'un agglomérat cellulaire mûrit,
- et à la suite de cette incubation, cet agglomérat cellulaire est respectivement soumis au moins à une seule charge de pression cyclique avec un façonnage mécanique qui suit.

2. Le procédé pour la fabrication de structures de tissu tridimensionnelles suivant la revendication 1 est **caractérisé en ce que** le premier pas des charges de pression cycliques mène à un agglomérat cellulaire qui est réduit jusqu'à 50% de son étendue avant le traitement par pression und que le façonnage mécanique est produit en ne pliant l'agglomérat cellulaire qu'une seule fois.

3. Le procédé pour la fabrication de structures de tissu tridimensionnelles suivant la revendication 2 est **caractérisé en ce que** la charge de pression est exécutée avec une fréquence de pression de 0,5 Hz et un temps de charge entre 15 et 25 secondes.

4. Le procédé pour la fabrication de structures de tissu tridimensionnelles suivant la revendication 1 est **caractérisé en ce que** le deuxième et les suivants pas des charges de pression cycliques sont respectivement interrompus par un façonnage mécanique intermédiaire en ne pliant l'agglomérat cellulaire qu'une seule fois.

5. Le procédé pour la fabrication de structures de tissu tridimensionnelles suivant la revendication 5 est **caractérisé en ce que** la charge de pression est exécutée avec une fréquence de pression de 0,5 Hz, et un temps de charge entre 15 et 25 secondes.

6. Le procédé suivant une ou plusieures des revendications précédentes est **caractérisé en ce que** la charge de pression cyclique avec un façonnage mécanique qui suit est interrompue après le cinquième jour de cultivation et l'agglomérat cellulaire est chargé d'une fréquence de pression de 0,5 Hz, un temps de charge entre 15 et 25 secondes avec une pression de 10 à 500 g/cm² jusqu'à 10 fois par jour et cela pendant une période de temps de jusqu'à 20 jours.

7. Structures de tissu tridimensionelles fabriquées selon le procédé d'une ou plusieures des revendications précédentes.
